# EUROPÄISCHE PATENTSCHRIFT

(11) **EP 1 488 817 B1**
(45) Veröffentlichungstag und Bekanntmachung des Hinweises auf die Patenterteilung: **01.05.2013**
(21) Anmeldenummer: 04020856.3
(22) Anmeldetag: 11.01.2002
(51) Int. Cl.: A61L 27/34, A61L 27/38, C12N 5/00, A61L 27/50, C08L 85/02, C08G 79/02

(54) **Polyphosphazen-haltige Substrate mit mikrostrukturierter Oberfläche**
Microstructured polyphosphazene-containing substrates
Substrats microstructurés contenant du polyphosphazène

(30) Priorität: 11.01.2001 DE 10100961
(43) Veröffentlichungstag der Anmeldung: 22.12.2004
(62) Teilanmeldung aus: 02715415.2
(73) Patentinhaber: CeloNova BioSciences Germany GmbH, 89077 Ulm (DE)
(72) Erfinder: Grunze, Michael, Prof. Dr., 69151 Neckargemünd (DE)
(74) Vertreter: Teipel, Stephan

(56) Entgegenhaltungen:
- WO-A-01/80919
- WO-A-99/45096
- LAURENCIN C T ET AL: "USE OF POLYPHOSPHAZENES FOR SKELETAL TISSUE REGENERATION" JOURNAL OF BIOMEDICAL MATERIALS RESEARCH, WILEY, NEW YORK, NY, US, Bd. 27, Nr. 7, Mai 1993 (1993-05), Seiten 963-973, XP008004104 ISSN: 0021-9304

## Beschreibung

Die vorliegende Erfindung betrifft Polyphosphazen-haltige Substrate mit einer formgebenden Oberfläche als Matrize zur Herstellung von in einen Säuger implantierbarer biologischer Materialien sowie Polyphosphazen-haltige Substrate mit mikrostrukturierter Oberfläche.

Die Züchtung von Zellen, insbesondere von Endothelzellen, mit dem Ziel künstliche Organe wachsen zu lassen, ist eine vergleichsweise neue Entwicklung in der Implantologie. Ein besonderer Vorteil dieser Technik ist die zu erwartende vollkommene Körperverträglichkeit von Implantaten, die auf diese Art hergestellt werden. Da *ex vivo* gezüchtete Zellansammlungen zunächst weder die Form noch eine gewünschte mechanische Stabilität des späteren Implantats, wie Organe, Arterien, etc., aufweisen, werden solche Implantate zunächst auf einem formgebenden Substrat vorgeformt. Gängige Substrate, auf denen solche Zellen gezüchtet werden und die das primäre Stützgerüst für ein solches Implantat darstellen, sind beispielsweise Polylactide, Polyethylenglycole, Polyurethane, Teflon sowie anorganische Substrate.

Aus dem Stand der Technik sind weiterhin eine Vielzahl von Materialien bekannt und untersucht, welche für die Herstellung von derartigen primären Stützgerüsten bzw. Stützsubstraten Verwendung finden. So ist beispielsweise in der WO 98/56312 eine expandierbare Hülle aus ε-PTFE bekannt, die auch für die Züchtung von künstlichen Adern verwendet werden kann. Andere Materialien für diese Verwendung sind in der EP-A-0 810 845, US-Patent 4,883,699 und US-Patent 4,911,691 beschrieben. Weitere Polymere für den genannten Zweck sind beispielsweise hydrolysiertes Polyacrylnitril (US-Patent 4,480,642), hydrophile Polyether (US-Patent 4,798,876), Polyurethandiacrylate (US-Patent 4,424,395). Ferner sind verschiedene Hydrogele bekannt, die als Beschichtung für diesen Zweck eingesetzt werden können. Die Reihe der potentiell anwendbaren Materialien kann weiterhin ergänzt werden durch Polyvinylpyrrolidone (PVP), Polyvinylalkohole (PVA), Polyethylenoxid (PEO) und Polyhydroxyethylmethacrylate p(HEMA). Ferner werden im Stand der Technik auch die Anwendung einer Reihe von Standardmaterialien wie Polyurethane, Polyethylene und Polypropylene als mögliche Werkstoffe für derartige Substrate beschrieben. Ebenso sind Mischungen dieser Werkstoffe untereinander bekannt. Eine Reihe weiterer Materialien ist aus der EP-A-0 804 909 bekannt.

Da die inhärenten Eigenschaften dieser Materialien unterschiedlich sind, kann davon ausgegangen werden, daß jedes dieser Materialien bzw. jeder dieser Werkstoffe besondere Eigenschaften für bestimmte Anwendungen in der Züchtung von künstlichen Implantaten aufweist. So ist beispielsweise PVA sehr gut in Flüssigkeit löslich und wird schneller resorbiert. Andere Materialien weisen eine gute Blutverträglichkeit auf. Wiederum andere Materialien sind besonders gut streckbar. Jedoch weisen bedauerlicherweise alle Materialien Nachteile in unterschiedlichen Bereichen auf. PVA zeigt beispielsweise keine besonders gute Blutverträglichkeit.

ε-PTFE ist beispielsweise sehr gut streckbar und weist auch eine gute Blutverträglichkeit auf, jedoch ist dieses Material ausgesprochen schwierig zu handhaben und die Herstellung von Stützsubstraten aus diesem Material erfordert eine Reihe von bestimmten Verarbeitungsschritten (vgl. WO 96/00103). Auch ist die so gewonnene Oberfläche des ε-PTFE-Stützsubstrats sehr porös, so daß Zellen sehr stark in dieses Material hineinwachsen und beim Abtrennen des gezüchteten Zellmaterials als Implantat von dem Stützsubstrat eine Beschädigung fast unvermeidlich ist. Bei anderen Materialien können elastische Eigenschaften, die für ein derartiges Stützsubstrat in einigen Fällen wichtig sind, nur durch die Zugabe von Weichmachern erreicht werden, welche die Blut- und Körperverträglichkeit vermindern und durch Ausschwemmen der "Weichmacher" zusätzlich eine nachteilige Beeinflussung der Zellzüchtung darstellen.

Die größten bekannten Schwierigkeiten, die bei einer Züchtung von Zellen für Implantate auftreten, sind somit zum einen Reaktionen mit dem Stützsubstrat oder mit den hierbei entstehenden Abbauprodukten. So ist beispielsweise bekannt, daß bei der Auflösung bzw. der Resorption und der Zersetzung einiger der aus dem Stand der Technik bekannten Stoffe Entzündungsreaktionen im Empfänger auftreten können (van der Gießen, Circulation Band 94, Nr. 7, 1996). Dies ergibt sich entweder durch die teilweise mangelhafte Verträglichkeit solcher Stützsubstrate oder aber durch die Reaktion auf Zersetzungsprodukte, welche bei der Zersetzung der genannten Stoffe entstehen. Weiterhin können Risse und Brüche in dem frisch gezüchteten Implantat auftreten, wenn das gezüchtete Implantat von dem Stützsubstrat entfernt werden soll. Dieser nachteilige Effekt liegt insbesondere darin begründet, daß die für das Implantat wachsenden Zellen eine sehr enge Verbindung mit dem Stützsubstrat, insbesondere mit beispielsweise Polylactit, eingehen und durch die Porenstruktur, die entweder durch das Auflösen oder aber die grundsätzliche Oberflächenbeschaffenheit des Stützsubstrats entsteht, so mit dem Stützsubstrat verwachsen, daß ein Entfernen ohne Beschädigung des gezüchteten Implantats praktisch nicht möglich ist.

Weiterhin spielt für die problemfreie Züchtung der genannten Implantate, insbesondere Zellen, das Verhalten gegenüber Bakterien und Proteinen, die sich auf den Oberflächen der Stützsubstrate ablagern, eine zentrale Rolle, da diese Ablagerungen wesentlich zu Entzündungen im Patienten und anderen Problemen beim Wachstum und der Züchtung der Zellen führen können.

Insbesondere bei der Herstellung von Gefäßimplantaten sind die genannten Risse, die beim Entfernen des gezüchteten Gefäßimplantats vom Stützsubstrat entstehen können, ein wesentlicher Aspekt. Diese Risse sind beispielsweise Ansatzpunkte für eine verstärkte Thrombenbildung im Empfänger bzw. Patienten und andere Ablagerungen (Proteine, Makrophagen etc.), welche nach Implantation zu einem Risiko für den Empfänger bzw. Patienten werden können.

Die WO 99/45096 offenbart ein Kulturgefäß aus Kunststoff, das für die Züchtung von Zell- und Gewebekulturen vorgesehen ist. Die Wachstumsfläche ist mikrostrukturiert und besitzt Erhebungen, deren Höhe kleiner als 110 µm ist.

Die WO 01/80919 offenbart ein antithrombogenes und körperverträgliches Polymer und dessen Verwendung zur Herstellung von Umhüllungen und Folien als Bestandteil therapeutischer Vorrichtungen zur Verhinderung von übermäßiger Zellproliferation.

Somit liegt der vorliegenden Erfindung die Aufgabe zugrunde, ein neues System zur Herstellung von Implantaten aus biologischem Material bereitzustellen, welches ein möglichst selektives Wachstum von gewünschten Zellen ermöglichen soll sowie ein im wesentlichen beschädigungsfreies Abtrennen des aus den gewünschten Zellen gezüchteten Implantats vom eingesetzten Stützsubstrat sicherstellen soll.

Diese Aufgabe wird durch die in den Ansprüchen gekennzeichneten Ausführungsformen gelöst. Insbesondere wird ein Substrat mit einer mikrostrukturierten Oberfläche bereitgestellt, welche mindestens teilweise ein biokompatibles Polymer mit der folgenden allgemeinen Formel (I) 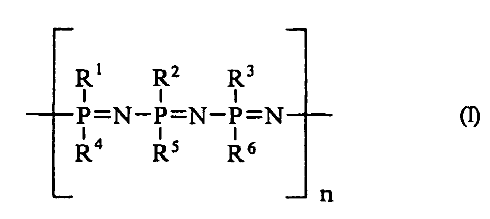 umfaßt, wobei n für 2 bis ∞ steht, R¹ bis R⁶ gleich oder unterschiedlich sind und einen Alkoxy-, Alkylsulfonyl-, Dialkylamino- oder Aryloxyreste oder einen Heterocycloalkyl- oder Heteroarylrest mit Stickstoff als Heteroatom bedeuten, und wobei die Oberflächenstruktur eine Größenordnung im Bereich von 10 nm bis 100 µm aufweist.

In einer bevorzugten Ausführungsform der vorliegenden Erfindung wird das biokompatible Polymer gemäß Formel (I) als Überzug auf dem Substrat zur Ausbildung der formgebenden Oberfläche bereitgestellt. In dieser erfindungsgemäßen Ausführungsform weist das verwendete Substrat keine besondere Beschränkung auf und kann jedes Material, wie Kunststoffe, Metalle, Metallegierungen und Keramiken, sein. Der biokompatible Überzug weist beispielsweise eine Dicke von etwa 1 nm bis etwa 1000 µm, vorzugsweise bis etwa 10 µm und besonders bevorzugt bis etwa 1 µm auf. In einer anderen bevorzugten Ausführungsform der vorliegenden Erfindung ist das Substrat mit einer formgebenden Oberfläche ein Formkörper aus dem biokompatiblen Polymer gemäß Formel (I).

Der Polymerisationsgrad des biokompatiblen Polymers gemäß Formel (I) liegt vorzugsweise in einem Bereich von 20 bis 200.000, mehr bevorzugt von 40 bis 100.000.

Vorzugsweise ist mindestens einer der Reste R¹ bis R⁶ im verwendeten Polymer ein Alkoxyrest, der mit mindestens einem Fluoratom substituiert ist.

Die Alkylreste in den Alkoxy-, Alkylsulfonyl- und Dialkylaminoreste sind beispielsweise gerad- oder verzweigtkettige Alkylreste mit 1 bis 20 Kohlenstoffatomen, wobei die Alkylreste beispielsweise mit mindestens einem Halogenatom, wie ein Fluoratom, substituiert sein können.

Beispiele für Alkoxyreste sind Methoxy-, Ethoxy-, Propoxy- und Butoxygruppen, die vorzugsweise mit mindestens einem Fluoratom substituiert sein können. Besonders bevorzugt ist die 2,2,2-Trifluoroethoxygruppe. Beispiele für Alkylsulfonylreste sind Methyl-, Ethyl-, Propyl- und Butylsulfonylgruppen. Beispiele für Dialkylaminoreste sind Dimethyl-, Diethyl-, Dipropyl- und Dibutylaminogruppen.

Der Arylrest im Aryloxyrest ist beispielsweise eine Verbindung mit einem oder mehreren aromatischen Ringsystemen, wobei der Arylrest beispielsweise mit mindestens einem vorstehend definierten Alkylrest substituiert sein kann. Beispiele für Aryloxyreste sind Phenoxy- und Naphthoxygruppen und Derivate davon.

Der Heterocycloalkylrest ist beispielsweise ein 3- bis 7- Atome enthaltendes Ringsystem, wobei mindestens ein Ringatom ein Stickstoffatom ist. Der Heterocycloalkylrest kann beispielsweise mit mindestens einem vorstehend definierten Alkylrest substituiert sein. Beispiele für Heterocycloalkylreste sind Piperidinyl-, Piperazinyl-, Pyrrolidinyl- und Morpholinylgruppen und Derivate davon. Der Heteroarylrest ist beispielsweise eine Verbindung mit einem oder mehreren aromatischen Ringsystemen, wobei mindestens ein Ringatom ein Stickstoffatom ist. Der Heteroarylrest kann beispielsweise mit mindestens einem vorstehend definierten Alkylrest substituiert sein. Beispiele für Heteroarylreste sind Pyrrolyl-, Pyridinyl-, Pyridinolyl-, Isochinolinyl- und Chinolingruppen, und Derivate davon.

In einer weiteren bevorzugten erfindungsgemäßen Ausführungsform ist zwischen der Oberfläche des Substrats und dem aus dem Polyphosphazenderivat aufgebauten, biokompatiblen Überzug eine Schicht angeordnet, die einen Adhäsionspromotor enthält.

Der Adhäsionspromotor bzw. Spacer enthält bevorzugt eine polare Endgruppe. Beispiele hierfür sind Hydroxy-, Carboxy-, Carboxyl-, Amino oder Nitrogruppen. Es können aber auch Endgruppen des Typs O-ED verwendet werden, wobei O-ED einen Alkoxy-, Alkylsulfonyl-, Dialkylamino- oder Aryloxyrest oder einen Heterocycloalkyl- oder Heteroarylrest mit Stickstoff als Heteroatom bedeutet und unterschiedlich, beispielsweise durch Halogenatome, insbesondere Fluor, substituiert sein kann.

Insbesondere kann der Adhäsionspromotor beispielsweise eine Silicium-organische Verbindung, vorzugsweise ein aminoterminiertes Silan bzw. basierend auf Aminosilan, aminoterminierte Alkene, nitroterminierte Alkene und Silane oder eine Alkylphosphonsäure sein. Besonders bevorzugt ist Aminopropyltrimethoxysilan.

Der Adhäsionspromotor verbessert insbesondere die Haftung des Überzugs auf der Oberfläche des Substrats durch Kopplung des Adhäsionspromotors an die Oberfläche des Substrats, beispielsweise über ionische und/oder kovalente Bindungen und durch weitere Kopplung des Adhäsionspromotors an das beschriebene Polymer der Formel (I) des Überzugs, beispielsweise über ionische und/oder kovalente Bindungen.

Der Begriff "biologisches Material" umfaßt beispielsweise eukaryontische Zellen, ein- oder mehrschichtige Zellverbände, Gewebe oder Zellbestandteile von Säuger-, insbesondere humanen Ursprungs. In einer bevorzugten Ausführungsform ist der Spender des biologischen Ausgangsmaterials identisch mit dem Empfänger für das implantierbare biologische Material. Beispiele für das biologische Ausgangsmaterial bzw. biologische Material sind Endothelzellen unterschiedlicher Herkunft (z.B. aus Haut, Vorhaut, Adern wie Aorta, Fettgewebe, Auge, Magennetz, Nabelschnur, Varizen oder ähnlichem), Epithelzellen unterschiedlicher Herkunft (z.B. aus Magen, Darm oder ähnlichem), Knochenzellen, Knorpelzellen, alle adhärenten Zellen oder Zellen, bei denen eine Adhärenz induzierbar ist, Zellverbände oder Gewebe (z.B. künstliche, in Kultur vorgezüchtete Haut oder ähnliche Gewebe), natürliche Gewebe sowie Proteine, Zuckermoleküle und Lipide. Unter Verwendung des Substrats mit formgebender Oberfläche können somit beispielsweise künstliche Organe, Adern, Knochen, Knorpel, Myelinscheiden etc. hergestellt werden.

In einem Verfahren zur Herstellung der vorstehend definierten Substrate mit einer formgebenden Oberfläche ist das Aufbringen eines Überzugs aus dem biokompatiblen Polymer gemäß Formel (I) auf die Oberfläche eines Formkörpers bzw. Stützsubstrats im Stand der Technik bekannt.

Beispielsweise kann die Herstellung der Substrate mit formgebender Oberfläche gemäß einer bevorzugten Ausführungsform im allgemeinen durch folgende Schritte ausgeführt werden:
(a) Eine Lösung, die mindestens eine Verbindung der allgemeinen Formel (I) in einer Konzentration von 0,1% - 99% enthält, wird in einem Lösungsmittel bereitgestellt, wobei dieses Lösungsmittel organisch und polar ist. Beispielsweise können hier als Lösungsmittel Ethylacetat, Aceton, THF, Toluol oder Xylole verwendet werden. Ebenso sind auch Mischungen dieser Lösungsmittel verwendbar oder können durch andere Lösungsmittel ergänzt werden. Diese Lösung wird auf ein Substrat gegeben, das keine oder ein geringe Adhäsion gegenüber dem Polymer zeigt, wie z.B. Glas, Silizium, verschiedene Keramiken oder andere entsprechende Werkstoffe, wie Polymere (PDMS, Teflon, PMMA, Polycarbonate oder Silicone). Die Oberflächen der aufgeführten Substrate können auch chemisch modifiziert sein, z.B. durch Einführung bestimmter funktioneller Gruppen (-NH₂, -OH, -COOH, -COH, -COOMe, -CF₃ usw.).
(b) Das Verdampfen des Lösungsmittels kann ohne weitere Maßnahmen ablaufen, jedoch wird im besten Fall die Konzentration des Lösungsmitteldampfes über dem Substrat kontrolliert eingestellt, wie auch der Druck und die Temperatur. Zu Beginn der ersten Trocknungsphase soll die Atmosphäre über dem beschichteten Substrat mit Lösungsmitteldampf gesättigt sein, wobei die Konzentration des Lösungsmitteldampfes dann über mehrere Stunden langsam reduziert wird. Die Temperatur kann von -30°C bis + 90°C variieren. Der Druck kann während der ersten Trocknungsphase einen Gradienten von Normaldruck bis Wasserstrahlvakuum (20 Torr) durchlaufen. Nach der ersten Trocknungsphase wird das beschichtete Substrat für eine bestimmte Zeit im Ölpumpenvakuum (0,1 Torr) weiter getrocknet.

Das mit dem biokompatiblen Polymer gemäß Formel (I) beschichtete Substrat kann dann, ohne oder nach entsprechender Sterilisation, direkt weiter verwendet werden. Je nach Konzentration der Polymerlösung und den angesprochenen Bedingungen während der ersten Trocknungsphase werden verschiedene Schichtdicken von 0,1 µm bis 300 µm oder dicker, vorzugsweise im Bereich von 0,5 µm bis 30 µm, und besonders bevorzugt um 5 µm erhalten.

Die vorliegende Erfindung betrifft ein Substrat mit einer mikrostrukturierten Oberfläche, welche mindestens teilweise ein biokompatibles Polymer gemäß Formel (I), wie vorstehend definiert, umfaßt, wobei die Oberflächenstrukturen eine Größenordnung im Bereich von 10 nm bis 100 µm, aufweisen. In einer bevorzugten Ausführungsform liegt das biokompatible Polymer als Überzug mit einer außenliegenden mikrostrukturierten Oberfläche auf der Substratoberfläche vor.

Die Strukturierung der Oberfläche unterliegt keiner besonderen Einschränkung. So können alle Strukturen hergestellt werden, die photolithographisch oder mit Elektronenstrahl oder mit Ionenstrahl oder mit dem Laser oder mittels anderer Techniken erzeugt werden können. Die Mikrostrukturierung der Oberfläche des Substrats bzw. des Überzugs kann auch durch "Schmelzstrukturieren" erhalten werden, wobei ein dünner Draht auf die Schmelztemperatur des biokompatiblen Polymers gebracht wird, welcher dann die gewünschte Struktur durch direkten Kontakt in die Oberfläche des Überzugs oder des Formkörpers einschmilzt.

Besondere Vorteile können durch diese Strukturierung dadurch erreicht werden, daß Strukturen in die Oberfläche des Überzugs bzw. des Substrats eingeprägt werden, die das Strömungsverhalten von Flüssigkeiten besonders günstig gestalten (z.B. Haifischhaut oder Lotoseffekt).

## Patentansprüche

1. Substrat mit einer mikrostrukturierten formgebenden Oberfläche als Matrize zur Herstellung von in einen Säuger implantierbarer biologischer Materialiern, welche mindestens teilweise ein biokompatibles Polymer mit der folgenden allgemeinen Formel (I) 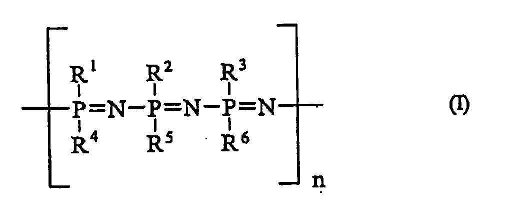 umfasst, wobei n für 2 bis ∞ steht, R¹ bis R⁶ gleich oder unterschiedlich sind und einen Alkoxy- Alkylsulfonyl-, Dialkylamino- oder Aryloxyrest oder einen Heterocycloalkyl- oder Heteroarylrest mit Stickstoff als Heteroatom bedeuten, und wobei die Oberflächenstrukturen eine Größenordnung im Bereich von 10 nm bis 100 µm aufweisen.

2. Substrat nach Anspruch 1, wobei das biokompatible Polymer als Überzug mit außenliegender mikrostrukturierter Oberfläche auf der Substratoberfläche vorliegt.

3. Substrat nach Anspruch 2, wobei zwischen der Oberfläche des Substrats und dem Überzug aus biokompatiblem Polymer eine Schicht angeordnet ist, die einen Adhäsionspromoter enthält.

4. Substrat nach Anspruch 3, wobei der Adhäsionspromoter eine polare Endgruppe enthaltende Verbindung, insbesondere eine Silizium-organische Verbindung, ist.

5. Substrat nach Anspruch 4, wobei die Silizium-organische Verbindung Aminopropyltrimethoxysilan ist.

6. Verwendung eines Substrats mit einer mikrostrukturierten Oberfläche, welche mindestens teilweise ein biokompatibles Polymer mit der folgenden allgemeinen Formel (I) 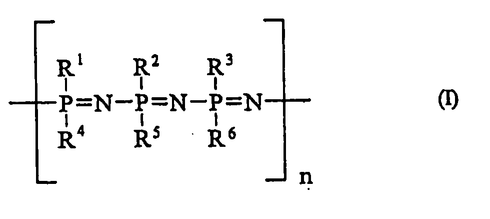 umfasst, wobei n für 2 bis ∞ steht, R¹ bis R⁶ gleich oder unterschiedlich sind und einen Alkoxy- Alkylsulfonyl-, Dialkylamino- oder Aryloxyrest oder einen Heterocycloalkyl- oder Heteroarylrest mit Stickstoff als Heteroatom bedeuten, und wobei die Oberflächenstrukturen eine Größenordnung im Bereich von 10 nm bis 100 µm aufweisen, als Substrat mit einer formgebenden Oberfläche als Matrize zur Herstellung von in einen Säuger implantierbarer biologischer Materialien.

7. Verwendung nach Anspruch 6, wobei das biokompatible Polymer als Überzug mit außenliegender mikrostrukturierter Oberfläche auf der Substratoberfläche vorliegt.

8. Verwendung nach Anspruch 7, wobei zwischen der Oberfläche des Substrats und dem Überzug aus biokompatiblem Polymer eine Schicht angeordnet ist, die einen Adhäsionspromoter enthält.

9. Verwendung nach Anspruch 8, wobei der Adhäsionspromoter eine polare Endgruppe enthaltende Verbindung, insbesondere eine Silizium-organische Verbindung, ist.

10. Verwendung nach Anspruch 9, wobei die Silizium-organische Verbindung Aminopropyltrimethoxysilan ist.

## Claims

1. A substrate having a micro-structured forming surface as a matrix for the production of biological materials implantable into a mammal, which comprises at least partially a biocompatible polymer of the following general formula (I) 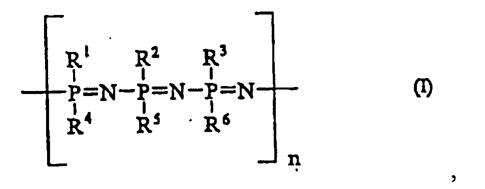 wherein n represents 2 to ∞, R¹ to R⁶ are the same or different and mean an alkoxy, alkylsulfonyl, dialkylamino, or aryloxy group or a heterocycloalkyl or heteroaryl group with nitrogen as heteroatom, and wherein the surface structures have an order in the range of 10nm to 100µm.

2. The substrate according to claim 1, wherein the biocompatible polymer is present as a coating with an exterior micro-structured surface on the substrate surface.

3. The substrate according to claim 2, wherein a layer containing an adhesion promoter is arranged between the surface of the substrate and the coating of biocompatible polymer.

4. The substrate according to claim 3, wherein the adhesion promoter is a polar end group-containing compound, in particular a silicon-organic compound.

5. The substrate according to claim 4, wherein the silicon-organic compound is aminopropyltrimethoxysilane.

6. Use of a substrate having a micro-structured surface which comprises at least partially a biocompatible polymer of the following general formula (I) 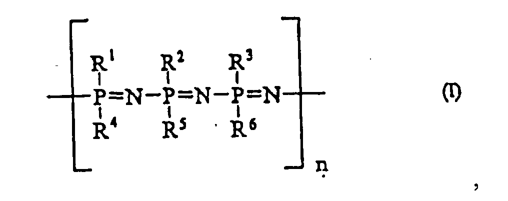 wherein n represents 2 to ∞, R¹⁰ R⁶ are the same or different and mean an alkoxy, alkylsulfonyl, dialkylamino, or aryloxy group or a heterocycloalkyl or heteroaryl group with nitrogen as heteroatom, and wherein the surface structures have an order in the range of 10nm to 100µm, as a substrate having a forming surface as a matrix for the production of biological materials implantable into a mammal.

7. The use according to claim 6, wherein the biocompatible polymer is present as a coating with an exterior micro-structured surface on the substrate surface.

8. The use according to claim 7, wherein a layer containing an adhesion promoter is arranged between the surface of the substrate and the coating of biocompatible polymer.

9. The use according to claim 8, wherein the adhesion promoter is a polar end group-containing compound, in particular a silicon-organic compound.

10. The use according to claim 9, wherein the silicon-organic compound is aminopropyltrimethoxysilane.

## Revendications

1. Substrat présentant une surface de façonnage microstructurée, comme matrice pour la fabrication de matériaux biologiques implantables chez un mammifère, qui présente au moins partiellement un polymère biocompatible répondant à la formule générale (I) suivante : 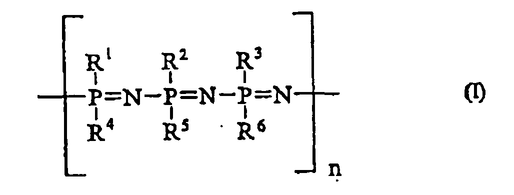 dans laquelle n vaut 2 à ∞, les radicaux R¹ à R⁶ sont identiques ou différents et représentent un résidu alcoxy, alkylsulfonyle, dialkylamino ou aryloxy, ou un résidu hétérocycloalkyle ou hétéroaryle avec de l'azote comme hétéroatome, et où les structures de surface présentent un ordre de grandeur dans la plage de 10 nm à 100 µm.

2. Substrat selon la revendication 1, dans lequel le polymère biocompatible est présent sur la surface du substrat sous la forme d'un revêtement ayant une surface microstructurée externe.

3. Substrat selon la revendication 2, dans lequel, entre la surface du substrat et le revêtement à base de polymère biocompatible, est aménagée une couche qui contient un promoteur d'adhérence.

4. Substrat selon la revendication 3, dans lequel le promoteur d'adhérence est un composé contenant des groupements terminaux polaires, en particulier un composé d'organosilicium.

5. Substrat selon la revendication 4, dans lequel le composé d'organosilicium est l'aminopropyl-triméthoxysilane.

6. Utilisation d'un substrat présentant une surface microstructurée, qui comprend au moins en partie un polymère biocompatible répondant à la formule générale (I) suivante : 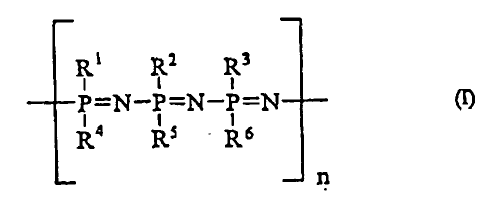 dans laquelle n vaut 2 à ∞, les radicaux R¹ à R⁶ sont identiques ou différents et représentent un résidu alcoxy, alkylsulfonyle, dialkylamino ou aryloxy, ou un résidu hétérocycloalkyle ou hétéroaryle avec de l'azote comme hétéroatome, et où les structures de surface présentent un ordre de grandeur dans la plage de 10 nm à 100 µm, comme substrat pourvu d'une surface de façonnage comme matrice pour la fabrication de matériaux biologiques implantables chez un mammifère.

7. Utilisation selon la revendication 6, dans laquelle le polymère biocompatible est présent à la surface du substrat sous la forme d'un revêtement ayant une surface microstructurée externe.

8. Utilisation selon la revendication 7, dans laquelle, entre la surface du substrat et le revêtement à base de polymère biocompatible, est aménagée une couche qui contient un promoteur d'adhérence.

9. Utilisation selon la revendication 8, dans laquelle le promoteur d'adhérence est un composé contenant des groupements terminaux polaires, en particulier un composé d'organosilicium.

10. Utilisation selon la revendication 9, dans laquelle le composé d'organosilicium est l'aminopropyl-triméthoxysilane.
